# EUROPÄISCHE PATENTANMELDUNG

(11) **EP 1 532 988 A2**
(43) Veröffentlichungstag der Anmeldung: **25.05.2005**
(21) Anmeldenummer: 05075349.0
(22) Anmeldetag: 19.08.2000
(51) Int. Cl.: A61K 49/00

(54) **Antikörper-Farbstoffkonjugate zur intraoperativen Tumorranddarstellung**

(30) Priorität: 24.09.1999 DE 19947559
(62) Teilanmeldung aus: 00954640.9
(71) Anmelder: Schering AG, 13342 Berlin (DE)
(72) Erfinder: Schirner, Michael, 13158 Berlin (DE); Licha, Kai, 14612 Fakensee (DE); Dinkelborg, Ludger, 13465 Berlin (DE)
(74) Vertreter: Kilger, Ute (DE)

(57) **Zusammenfassung**

Es werden Antikörper-Farbstoffkonjugate, die geeignet sind an Strukturen neugebildeter Gefäße zu binden und deren Verwendung zur intraoperativen Darstellung der pathologischen Angiogenese beschrieben.

## Beschreibung

Die vorliegende Erfindung betrifft Antikörper-Farbstoffkonjugate, die geeignet sind an Strukturen neugebildeter Gefäße zu binden und deren Verwendung zur intraoperativen Darstellung der pathologischen Angiogenese.

Im erwachsenen Organismus findet bis auf wenige Ausnahmen (z. B. der Zyklus der gebärfähigen Frau) keine Neubildung von Gefäßen statt. Die Neubildung von Gefäßen ist jedoch bei vielen Erkrankungen zu beobachten. Der hier stattfindende Prozeß der Gefäßneubildung wird als Angiogenese bezeichnet und findet als Antwort auf bestimmte Signale statt.

Die Angiogenese ist ein Prozeß, der im Randbereich eines Krankheitsherdes bevorzugt stattfindet. Aus dem Zentrum des Krankheitsherdes werden Faktoren freigesetzt, die zum Randbereich des Krankheitsherdes diffundieren. Diese Faktoren werden auch als Angiogenesestimulatoren bezeichnet. Erreichen diese Angiogenesestimulatoren das gesunde Gewebe im Randbereich eines Krankheitsherdes, werden bisher nicht in den Krankheitsherd einbezogene Gefäße stimuliert, neue Gefäßknospen zu bilden. Die von diesen Gefäßknospen auswachsenden Gefäße bilden ein neues Kapillargefäßnetz im Randbereich des Krankheitsherdes. Durch diesen Prozeß kann immer eine adäquate Nährstoffversorgung für den Krankheitsherd sichergestellt werden. Als besonders wichtig hat sich herausgestellt, daß das Wachstum von Tumoren und deren Metastasen von der Fähigkeit abhängt, die Angiogenese zu induzieren.

Die chirurgische Therapie ist heute eine Standardmaßnahme zur Behandlung von lokalisierten Krankheitsherden. Große Bedeutung hat sie bei der Tumorbehandlung erlangt. Es hat sich aber herausgestellt, daß trotz verbesserter chirurgischer Techniken die Zahl der lokalen Rezidive beträchtlich ist, da die anatomischen Gegebenheiten im menschlichen Organismus nur selten eine großräumige Entfernung der Krankheitsherde zulassen. In vielen Organen (z. B. im Gehirn) muß auf ein großräumiges Entfernen verzichtet werden, um gesundes Gewebe zu erhalten. Das Risiko der Verletzung gesunder Organe steigt mit der Radikalität des chirurgischen Eingriffs.

Histologische Untersuchungen des Tumorrandbereiches nach erfolgter chirurgischer Tumorentfernung haben jedoch gezeigt, daß eine Vielzahl von Tumoren nicht vollständig entfernt werden können und Tumorreste im Körper verbleiben. Von diesen Tumorresten kann weiteres Tumorwachstum und auch die Tumormetastasierung ausgehen. Ein Verfahren, daß die Grenzen eines Krankheitsprozesses zum gesunden Gewebe während der chirurgischen Behandlung exakt darstellt, würde es erlauben, Krankheitsherde vollständig zu entfernen und das gesunde Gewebe weitgehend zu schonen.

Farbstoffe zur Darstellung von Krankheitsherden sind bereits bekannt (Poon WS et al., J Neurosurgery (1992) 76: 679-686, Haglund MM et al., Neurosurgery (1996) 38: 308-317). Sie werden vorzugsweise von Tumorzellen direkt aufgenommen oder reichern sich unspezifisch im extrazellulären Raum der Tumoren an. Da der Mechanismus der Anreicherung auch im gesunden Gewebe nachweisbar ist, ist die Spezifität und Empfindlichkeit der verwendeten Substanzen gering.

Verbindungen, die für die intraoperative Abgrenzung der Krankheitsherde durch selektive Darstellung des Randbereiches eines Krankheitsherdes verwendet werden können, sind bisher nicht bekannt.

Die Angiogenese findet im Randbereich von Krankheitsherden bevorzugt statt. Durch Darstellung der Angiogenese kann die Grenze zum gesunden Gewebe dargestellt werden. Antikörper zum Nachweis der Angiogenese im Krankheitsherd sind bereits bekannt und werden zur Darstellung von neugebildeten Gefäßen im histologischen Gewebeschnitt, zum Nachweis verschiedener Proteine im Krankheitsherd oder als Trägermoleküle für therapeutische Substanzen verwendet.

Nicht bekannt sind jedoch Antikörper in Kombination mit Farbstoffen, sogenannte Antikörper-Farbstoffkonjugate, die für die intraoperative Abgrenzung der Krankheitsherde durch selektive Darstellung des Randbereichs eines Krankheitsherdes zum Einsatz kommen können.
Aufgabe der vorliegenden Erfindung ist es daher, Antikörper-Farbstoffkonjugate für die intraoperative Tumorranddarstellung bereitszustellen. Die Antikörper der erfindungsgemäßen Antikörper-Farbstoffkonjugate sind gegen Strukturen gerichtet, die spezifisch für den Prozeß der Angiogenese sind. Die erfindungsgemäßen Antikörper-Farbstoffkonjugate umfassen Farbstoffe, die durch ihre Anreicherung eine optisch Darzustellung ermöglichen.
Da die Angiogenese im Randbereich eines Krankheitsherdes am stärksten ausgebildet ist, kommt es hier zum größten optischen Signal.
Die erfindungsgemäßen Antikörper-Farbstoffkonjugate sind somit geeignet, die Grenze eines Krankheitsherdes, den sogenannten Randbereich, zum gesunden Gewebe durch intraoperative, optische Diagnostik darzustellen. Hierdurch wird es ermöglicht, den Krankheitsherd bei weitgehender Schonung des gesunden Gewebes vollständig zu entfernen.

Es sind Antikörper bekannt, die gegen Moleküle gerichtet sind, die im angiogenetisch aktiven Gewebe stark exprimiert und im angrenzenden Gewebe nur auf sehr geringem Niveau exprimiert sind (WO 96/01653).

Von besonderem Interesse in den Antikörper-Farbstoffkonjugaten sind Antikörper, die gegen die Rezeptoren für vaskuläre Wachstumsfaktoren gerichtet sind, Rezeptoren auf Endothelzellen, an die Entzündungsmediatoren binden, Rezeptoren auf Endothelzellen, an die Matrixmoleküle binden und Matrixproteine, die spezifisch bei der Gefäßneubildung exprimiert werden (Brekken et al., Cancer Res. (1998) 58: 1952-9 und Schold SC Jr et al., Invest. Radiol. (1993) 28: 488-96).

Bevorzugt sind Antikörper oder Antikörperfragmente, die gegen das Matrixprotein EDB-Fibronektin gerichtet sind. EDB-Fibronektin (EDBFN), auch als onkofetales Fibronektin bekannt, ist eine Splicevariante des Fibronektins, das sich spezifisch um neugebildete Gefäße im Prozeß der Angiogenese bildet. Der besondere Vorteil von Antikörpern gegen das EDB-Fibronektin besteht darin, daß es durch intraoperative Verwundung bei der Entfernung des Krankheitsherdes zu keiner Neubildung des EDB-Fibronektins im gesunden Gewebe kommt. Hierdurch bleibt die Spezifität während des chirurgischen Eingriffs erhalten. Antikörper gegen Wachstumsfaktorrezeptoren oder Entzündungsmediatoren auf der Endothelzelle, die ebenfalls spezifisch im Tumorrandbereich exprimiert werden, können aber während des chirurgischen Eingriffs auch im gesunden Gewebe in der Nähe des Krankheitsherdes neugebildet werden.

Besonders bevorzugt im erfinderischen Antikörper-Farbstoffkonjugat sind die Antikörper L19 und E8 gegen das EDB-Fibronektin (Viti F et al, Cancer Res (1999) 59: 347-352).
Solche Antikörper-Farbstoffkonjugate sind ebenfalls Gegenstand der vorliegenden Erfindung.

Die bekannten Antikörper werden mit Farbstoffen konjugiert, deren Anreicherung im Gewebe optisch detektiert werden kann und die intraoperativen Abgrenzung des Randbezirkes eines Krankheitsherdes ermöglicht.

Der Vorteil der erfindungsgemäßen Antikörper-Farbstoffkonjugate besteht nun darin, daß diese für eine selektive Fluoreszenzanfärbung von Geweben in neoangiogenetischem Stadium zur Anwendung kommen können. Die Fluoreszenzanfärbung ist tumorspezifisch und liefert ein Fluoreszenzsignal, daß in hohem Signal-zu-Untergrund-Verhältnis detektiert werden kann.

Es sind auch Antikörper-Farbstoffkonjugate für die Fluoreszenzbildgebung zum Zwecke der perkutanen, nicht-invasiven Tumordarstellung bekannt (Neri D et al., Nature Biotechnology (1997) 15: 1271-1275).

Nicht bekannt jedoch sind Antikörper-Farbstoffkonjugate, die sich im Randbereich eines Krankheitsherdes bevorzugt anreichern.

Es sind auch Protein-Farbstoffkonjugate zur intraoperativen Tumordarstellung bekannt.
Nachteilig an diesen Konjugaten ist, daß insbesondere hypoxische als auch metabolisch unterversorgte Tumorzellen die Konjugate aufnehmen. Da das Gewebe im Randbereich von Tumoren aber gut vaskularisiert ist und hierdurch die Zellen ausreichend mit Sauerstoff und Nährstoffen versorgt sind, gelingt gerade hierdurch keine ausreichende Anreicherung der bekannten Protein-Farbstoffkonjugate.

Dagegen sind die erfindungsgemäßen Antikörper-Farbstoffkonjugate von dem metabolischen Zustand des Krankheitsherdes weitgehend unabhängig.

Obwohl die optische Erfassung der Grenzen eines Krankheitsherdes auf unterschiedliche Weise erfolgen kann, wird generell die Erfassung der durch entsprechendes Anregungslicht induzierten, farbstoffspezifischen Fluoreszenzstrahlung bevorzugt. Je nach Emissionswellenlänge kann dabei die Fluoreszenz direkt makroskopisch oder mikroskopisch visuell erfaßt werden und gegebenenfalls dabei gleichzeitig durch bildgebende Detektionssysteme digital aufgezeichnet und auf einem Bildschirm dargestellt werden.

Visuell erfaßbar ist Fluoreszenzstrahlung des Spektralbereiches 400 bis 650 nm. Besonders bevorzugt ist eine Wellenlänge von 450 bis 600 nm. Der besondere Vorteil der Verwendung des sichtbaren Bereiches des Lichtes besteht darin, daß die Detektion der Fluoreszenz durch geringen technischen Aufwand möglich ist. Anregungslicht, das durch geeignete Laser oder Laserdioden erzeugt wird, wird in einen Lichtleiter eingekoppelt und über diesen an das zu diagnostizierende Areal herangeführt. Die Durchführung der introperativen Tumorranderkennung erfolgt durch großflächige Bestrahlung des Areals. Durch einen Filter (z. B. eine Filterbrille, die durch die untersuchende Person getragen wird) wird das reflektierte Anregungslicht abgeblockt und nur die farbstoffspezifische Fluoreszenz beobachtet (makroskopische Beobachtung). Alternativ kann die Erfassung der Fluoreszenz durch ein Operationsmikroskop erfolgen (mikroskopische Beobachtung). Durch die geringe Eindringtiefe von VIS-Licht in Gewebe (wenige Millimeter) können auf diese Weise oberflächlich lokalisierte Gefäßneubildungen erfaßt werden.

Ein weitererer Vorteil des Spektralbereiches des sichtbaren Lichtes besteht in der geringen Eindringtiefe in das Gewebe und Emission aus dem Gewebe. Hierduch wird das detektierbare Signal nicht durch Signale aus tieferen Gewebsanteilen verfälscht und kann den oberflächlich sichtbaren Gewebsstrukturen genau zugeordnet werden.

Gegenstand der vorliegenden Erfindung sind somit auch Antikörper-Farbstoffkonjugate, deren Farbstoffe im sichtbaren Spektralbereich des Lichtes ein optisches Signal induzieren.
Die Verwendung von Antikörper-Farbstoffkonjugaten mit Farbstoffen, die im Spektralbereich des Nahinfrarotlichtes (NIR; 600 - 900 nm) absorbieren, ermöglicht dagegen die Erkennung von Gefäßneubildung in tieferen Gewebeschichten (bis zu 1 cm), da NIR-Licht schwächer von Gewebe absorbiert wird und daher eine größere Gewebeeindringtiefe besitzt. Die Beobachtung der Fluoreszenz ist visuell nicht möglich und kann durch CCD-Kameras (charge coupled device-Kamera) erfolgen, die über dem interessierenden Gewebeareal plaziert sind. Sowohl die makroskopische als auch mikroskopische Erfassung ist möglich. Der Vorteil der Verwendung von Farbstoffen in den Antikörper-Farbstoffkonjugaten, die im NIR-Spektralbereich absorbieren und fluoreszieren, kommt dann zum tragen, wenn eine Beurteilung verdeckter Areale (z. B. durch Blut) erforderlich ist.

Aus photophysikalischer Sicht sind für die Antikörper-Farbstoffkonjugate solche Farbstoffe geeignet, die ein Absorptionsmaximum innerhalb des Spektralbereiches von 400 bis 800 nm und mindestens ein Fluoreszenzmaximum innerhalb 500 bis 900 nm besitzen.

Ebenfalls Gegenstand der vorliegenden Erfindung sind Antikörper-Farbstoffkonjugate, die dadurch gekennzeichnet sind, daß der Farbstoff erst unter Verwendung eines definierten Wellenlängenbereiches des sichtbaren oder nahinfraroten Lichtes ein Fluoreszenzsignal induziert.

Antikörper-Farbstoffkonjugate umfassend Farbstoffe mit visuell erfaßbarer Fluoreszenz, sind beispielsweise solche aus folgenden Klassen:
Fluorescein, Fluorescein-isothiocyanat, Carboxyfluorescein oder Calcein, Tetrabromfluoresceine oder Eosine, Tetraiodfluoresceine oder Erythrosine, Difluorofluorescein, wie z. B. Oregon Green™ 488, Oregon Green™ 500 oder Oregon Green™ 514, Carboxyrhodol (Rhodol Green™)-Farbstoffe (US 5,227,487; US 5,442,045), Carboxyrhodamin-Farbstoffe (z. B. Rhodamine Green™ Dyes) (US 5,366,860),
4,4-Difluoro-4-bora-3a,4a-diaza-indacene, wie z. B. Bodipy FL, Bodipy 493/503 oder Bodipy 530/550 und Derivate davon (US 4,774,339, US 5,187,288, US 5,248,782, US 5,433,896, US 5,451,663),
Cyaninfarbstoffe, insbesondere Carbocyanine und Merocyanine, Coumarinfarbstoffe, wie z. B. 7-Amino-4-methylcoumarin, Metallkomplexe von DTPA oder Tetraaza-macrozyclen (Cyclen, Pyclen) mit Terbium oder Europium oder Tetrapyrrolfarbstoffe, insbesondere Porphyrine.

Antikörper-Farbstoffkonjugate umfassend Nahinfrarotfarbstoffe, sind beispielsweise solche aus folgenden Klassen:
Polymethinfarbstoffe, wie Dicarbocyanin-, Tricarbocyanin-, Merocyanin- und Oxonolfarbstoffe (WO 96/ 17628),
Rhodaminfarbstoffe,
Phenoxazin- oder Phenothiazinfarbstoffe,
Tetrapyrrolfarbstoffe, insbesondere Benzoporphyrine, Chorine und Phthalocyanine.
Bevorzugte Nahinfrarotfarbstoffe in den Antikörper-Farbstoffkonjugaten sind die Cyaninfarbstoffe mit Absorptionsmaxima zwischen 700 und 800 nm, insbesondere Indodi- und Indotricarbocyanine.

Generell bevorzugt sind Farbstoffe in den Antikörper-Farbstoffkonjugaten aus o. g. Klassen, die eine oder mehrere Carboxylgruppen besitzen, welche nach chemischer Aktivierung an Aminogruppen von Antikörpern oder Antikörperfragmenten gekoppelt werden. Auch sind solche Derivate bevorzugt, die Maleimido- oder Bromalkylreste enthalten, so daß eine kovalente Kopplung an die Sulfhydrylgruppe der Aminosäure Cystein erfolgt.
Weiterhin sind Farbstoffe bevorzugt, die Isothiocyanat-Gruppen besitzen, welche ebenfalls mit Aminogruppen reagieren.
Darüber hinaus müssen die Farbstoffe in den Antikörper-Farbstoffkonjugaten eine hohe Photostabilität besitzen und unter Bestrahlung mit Licht nicht ausbleichen (Photobleaching), um innerhalb des Untersuchungszeitraums ein konstantes Signal zu gewährleisten.

Gegenstand der vorliegenden Erfindung sind somit Antikörper-Farbstoffkonjugate, die sich im Randbereich des Zellgewebes eines Krankheitsherdes bevorzugt anreichern und damit den Randbereich des Krankheitsherdes optisch darstellbar machen.

Insbesondere Gegenstand der vorliegenden Erfindung sind Antikörper-Farbstoffkonjugate der allgemeinen Formel I

B-(F)ₙ (I),

in der
- B: für einen Antikörper oder ein Antikörperfragment mit hoher Bindung an ED-BFN steht,
- F: für einen Farbstoff aus der Klasse der Coumarine, der Fluoresceine, Carboxyfluoresceine, der Difluorofluoresceine, der Tetrabromfluoresceine, der Tetraiodfluoresceine, der Rhodamine, der Carboxyrhodamine, der Craboxyrhodole, der 4,4-Difluoro-4-bora-3a, 4a-diaza-indacene, der Polymethinfarbstoffe oder der Tetrapyrrolfarbstoffe, oder der Terbium- oder Europiumkomplexe mit DTPA oder Cyclen und dessen Derivaten steht
und
- n: für 1 bis 5 steht, bedeuten.

Besonders bevorzugt und damit ebenfalls Gegenstand der vorliegenden Erfindung sind Antikörper-Farbstoffkonjugate, deren Farbstoff ein Cyaninfarbstoff, ein Merocyaninfarbstoff, ein Oxonolfarbstoff, ein Styrylfarbstoff oder ein Squariliumfarbstoff ist.

Insbesondere bevorzugt und damit ebenfalls Gegenstand der vorliegenden Erfindung sind Antikörper-Farbstoffkonjugate, in denen der Farbstoffanteil ein Cyaninfarbstoff, insbesondere ein Carbocyanin, Dicarbocyanin oder Tricarbocyanin ist.

Die Erfindung betrifft somit insbesondere solche Antikörper-Farbstoffkonjugate, in denen der Farbstoff -(F)ₙ der allgemeinen Formel I ein Cyaninfarbstoff der allgemeinen Formel II 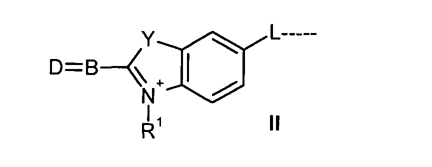 ist, in der
- D: für einen Rest III oder IV 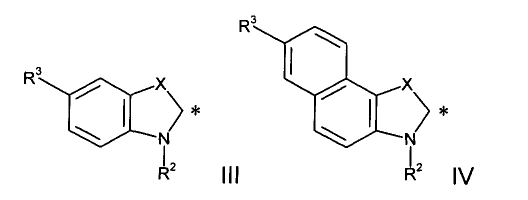 steht, wobei die mit einem Stern markierte Position die Verknüpfungsstelle mit dem
Rest B bedeutet, und
- B: für die Gruppe V, VI, VII, VIII oder IX 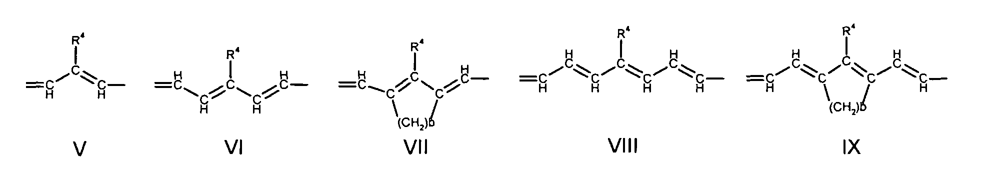 stehen kann, in denen
- R¹ und R²: C₁-C₄-Sulfoalkyl, eine gesättigte oder ungesättigte, verzweigte oder lineare C₁-C₅₀-Alkylkette bedeutet, die gegebenfalls mit bis zu 15 Sauerstoffatomen, und/oder mit bis zu 3 Carbonylgruppen, und/oder mit bis zu 5 Hydroxygruppen substituiert sein kann,
- R³: für die Gruppe -COOE¹, -CONE¹E², -NHCOE¹, -NHCONHE¹, -NE¹E², -OE¹, -OSO₃E¹, -SO₃E¹, -SO₂NHE¹ oder -E¹ steht, wobei
- E¹ und E²: unabhängig voneinander für ein Wasserstoffatom, C₁-C₄-Sulfoalkyl, gesättigtes oder ungesättigtes, verzweigtes oder geradkettiges C₁-C₅₀-Alkyl steht, das gegebenfalls mit bis zu 15 Sauerstoffatomen, und/oder bis zu 3 Carbonylgruppen unterbrochen, und/oder mit bis zu 5 Hydroxygruppen substituiert sein kann,
- R⁴: für ein Wasserstoffatom oder ein Fluor- Chlor, Brom- oder lodatom steht,
- b: für 2 oder 3 steht,
- X und Y: unabhängig voneinander für Sauerstoff, Schwefel oder die Gruppe =C(CH₃)₂ oder -(CH=CH)- steht,
und
- L: für eine direkte Bindung oder einen Linker, der eine geradkettige oder verzweigte Kohlenstoffkette mit bis zu 20 Kohlenstoffatomen, welche mit einer oder mehreren -OH, -COOH, SO₃-Gruppen substituiert, und/ oder gegebenenfalls ein oder mehrfach durch eine -O-, -S-, -CO-, -CS-,-CONH-, -NHCO-, -NHCSNH-, -SO₂-, PO₄⁻ oder eine -NH-Gruppen oder einen Arylring unterbrochen sein kann, steht.

Die erfindungsgemäßen Antikörper-Farbstoffkonjugate können entweder alleine oder in Formulierung als Arzneimittel zur Anwendung kommen.

Zur Verwendung der Antikörper-Farbstoffkonjugate als Arzneimittel werden diese in die Form eines pharmazeutischen Präparats gebracht, das neben dem Antikörper-Farbstoffkonjugat für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungsmittel, Stabilisierungsmittel, Netzmittel oder Emulgatoren, Salze zur Veränderung des osmotischen Drucks oder Puffer.

Für die parenterale Anwendung sind Injektionslösungen oder Suspensionen, insbesondere wässrige Lösungen der Antikörper-Farbstoffkonjugate geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden. Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.
Die Dosierung der Antikörper-Farbstoffkonjugate kann je nach Verabfolgungsweg, Alter und Gewicht des Patienten, Art und Schwere der zu behandelnden Erkrankung und ähnlichen Faktoren variieren. Die anwendbare Dosis der Antikörper-Farbstoffkonjugate zur Erkennung der Grenzbereiche beträgt 0,5-1000 mg, vorzugsweise 50-200 mg, wobei die Dosis als einmal zu verabreichende Einzeldosis oder unterteilt in 2 oder mehreren Tagesdosen gegeben werden kann.

Die oben beschrieben Formulierungen und Darreichungsformen sind ebenfalls Gegenstand der vorliegenden Erfindung.
Somit betrifft die Erfindung auch pharmazeutische Mittel, die ein oder merere Antikörper-Farbstoffkonjugate umfassen, zur intraoperativen Darstellung der Randbereiche eines Krankheitsherdes, wobei die pharmazeutischen Mittel entweder alleine oder in Mischung mit geeigneten Lösungsmitteln, Puffern und/oder Trägerstoffen zur Anwendung kommen.

Die erfindungsgemäßen Antikörper-Farbstoffkonjugate kommen bei der chirurgischen Behandlung von angiogeneseabhängigen Erkrankungen, wie malignen Tumoren und deren Metastasen, benignen Tumoren, präkanzeröse Gewebsveränderungen, Endometriose, Hämangiomen, extrauterinen Schwangerschaften zum Einsatz.

Ebenfalls Gegenstand der vorliegenden Erfindung ist die Verwendung der Antikörper-Farbstoffkonjugate und Mittel zur intraoperative Darstellung von Krankheitsherden, insbesondere zur mikro- und makroskopischen, intraoperativen Darstellung der Randbereiche eines Krankheitsherdes, sowie die Verwendung der Antikörper-Farbstoffkonjugate zur Herstellung eines Mittels für chirurgische Behandlungen von angiogeneseabhängigen Erkrankungen, wie malignen Tumoren und deren Metastasen, benignen Tumoren, präkanzeröse Gewebsveränderungen, Endometriose, Hämangiomen und extrauterinen Schwangerschaften.

### Herstellung der Farbstoffe

Die Herstellung der Farbstoffe erfolgt nach literaturbekannten Methoden. Geeignete Farbstoffe für die Herstellung der Antikörper-Farbstoffkonjugate sind Farbstoffe Carboxylgruppen oder Isothiocyanatgruppen zur kovalenten Kopplung an Aminogruppen des Antikörpers. Besonders bevorzugt sind hierbei Cyaninfarbstoffe (Mujumdar SR et al. (1996) 7: 356-362; Flanagan JH et al. (1997) 8: 751-756 und Licha K et al. (1996) Proc SPIE Vol 2927, 192-198).

Die Farbstoffe mit Carboxylgruppen werden zunächst durch Überführung in einen reaktiven Ester (z. B. N-Hydroxysuccinimidester) nach an sich bekannten Methoden aktiviert. Farbstoffe mit Isothiocyanatgruppen können direkt eingesetzt werden. Die reaktiven Derivate werden dann in Pufferlösung oder Gemischen aus organischem Lösungsmittel (z. B. Dimethylformamid (DMF) oder Dimethylsulfoxid (DMSO)) und Pufferlösung mit dem Antikörper zur Reaktion gebracht. Dabei wird ein 3 bis 100-facher molarer Überschuß an Farbstoff verwendet. Der nicht reagierte Anteil wird nach beendeter Reaktion durch Ultrafiltration und/oder Chromatographie abgetrennt.

In analoger Verfahrensweise wird auch folgender Farbstoff hergestellt:

### Herstellungsbeispiel 1

### Bis-1,1'-(4-sulfobutyl)indocarbocyanin-5-carbonsäure-N-hydroxysuccinimidester

Die Herstellung von Bis-1,1'-(4-sulfobutyl)indocarbocyanin-5-carbonsäure erfolgt ausgehend von 1-(4-Sulfobutyl)-2,3,3-trimethyl-3H-indolenin und 1-(4-Sulfobutyl)-2,3,3-trimethyl-5-carboxy-3H-indolenin (Cytometry 10, 11-19, 1989, Talanta 39, 505-510, 1992) in Anlehnung an literaturbekannte Methoden.
Zur Überführung in den N-Hydroxysuccinimidester wird 0,1 mmol Farbstoff (67 mg in 10 ml DMF) mit jeweils 0,5 mmol N-Hydroxysuccinimid und Dicyclohexylcarbodiimid (DCC) versetzt und 24 h bei Raumtemperatur gerührt. Nach Zugabe von 50 ml Ether wird der ausgefallene Feststoff abfiltriert, erneut je zweimal in wenig DDF gelöst und mit Ether gefällt und schließlich im Vakuum getrocknet (Ausbeute 89%).

### Herstellung des Antikörper-Farbstoffkonjugats

### Herstellung eines Bis-1,1'-(4-sulfobutyl)indocarbocyanin-Konjugates mit L19-Antikörper

Der Antikörper L19 (1 mg in 1 ml Natriumacetat-Puffer 50 mM, pH 8,2)) wird mit N-Hydroxysuccinimidester (75 µmol einer Lösung von 4 mg/ml in DMSO) versetzt und 2 h bei Raumtemperatur gerührt. Die Aufreinigung erfolgt mittels Gelfiltration über PD10-Kartuschen (Pharmacia) und Aufkonzentration mittels Centricon-10 tubes (Amicon) unter Erhalt einer Lösung von ca. 1 mg/ml Antikörper.
Absorptionsmaximum: 555 nm
Fluoreszenzmaximum: 582 nm.

Das nachfolgenden Beispiel erläutern die biologische Anwendbarkeit der erfindungsgemäßen Antikörper-Farbstoffkonjugate ohne diese auf die Anwendungsbeispiele zu beschränken.

### Anwendungsbeispiel 1

### In-vivo-Fluoreszenzbildgebung an tumortragenden Nacktmäusen und mikroskopische Ex-vivo-Untersuchung des Tumorgewebes

Die bildgebenden Eigenschaften der erfindungsgemäßen Verbindungen wurden in vivo nach Injektion in tumortragenden Nacktmäusen untersucht. Dazu wird 0,1 _{N}mol/kg bis 2 µmol/kg der Substanz intravenös appliziert und die Anreicherung in der Tumorregion in einem Zeitraum von 0 bis 48 Stunden beobachtet. Die Fluoreszenz der Substanzen wird durch Bestrahlung der Tiere mit Licht entsprechender Wellenlänge, das mit einem Laser (Diodenlaser, Festkörperlaser) monochromatisch erzeugt wird oder durch Filter aus der polychromatischen Emission einer Hg- oder Xe-Lampe herausgefiltert wird, induziert. Im Fall der im Herstellungsbeispiel 1 beschriebenen Verbindung wird aus einem Nd:YAG Laser Licht der Wellenlänge 540 nm zur Anregung zur Ausleuchtung des Versuchstieres verwendet und die Fluoreszenzstrahlung bei einer Wellenlänge von >580 nm durch eine intensivierte CCD-Kamera unter Erhalt von Ganzkörperfluoreszenzaufnahmen detektiert. Parallel wird die Fluoreszenz visuell und photographisch erfasst. Aus dem Tumormaterial werden Schnitte angefertigt und mikroskopisch untersucht (Zeiss Axiovert Mikroskop mit Cy3-Filtersatz).
Nach Injektion von 1 µmol/kg des im Herstellungsbeispiel genannten Antikörper-Farbstoffkonjugates in F9-Teratokarzinomtragenden Nacktmäusen konnte nach 4 h ein erhöhtes Fluoreszenzsignal im Vergleich zu Normalgewebe anhand von Ganzkörperfluoreszenzaufnahmen detektiert werden.
Nach Präparation der Haut und der obersten Gewebeschichten des Tumors kann die Fluoreszenz den Randbereichen des Tumors zugeordnet werden. Die mikroskopische Beurteilung von Tumorschnitten ergibt eine erhöhte Fluoreszenz, die mit Blutgefäßen des Tumorrandbereiches korreliert.

## Patentansprüche

1. Antikörper-Farbstoffkonjugate, die sich im Randbereich des Zellgewebes eines Krankheitsherdes bevorzugt anreichern und damit den Randbereich des Krankheitsherdes optisch darstellbar machen, **dadurch gekennzeichnet, dass** der Farbstoff eine Verbindung der allgemeinen Formel I
B-(F)ₙ (I),
in der
B für einen Antikörper oder ein Antikörperfragment mit hoher Bindung an EDB- Fibronektin steht,
F für einen Farbstoff aus der Klasse der Coumarine, der Fluoresceine, Carboxyfluoresceine, der Difluorofluoresceine, der
Tetrabromfluoresceine, der
Tetraiodfluoresceine, der Rhodamine, der Carboxyrhodamine, der
Carboxyrhodole, der 4,4-Difluoro-4-bora-3a,4a-diaza-indacene, der
Polymethinfarbstoffe oder der Tetrapyrrolfarbstoffe, oder der Terbiumoder
Europiumkomplexe mit DTPA oder Cyclen und dessen Derivaten steht und
n für 1 bis 5 steht, ist.

2. Antikörper-Farbstoffkonjugate gemäß Anspruch 1, **dadurch gekennzeichnet, dass** der Farbstoff ein Cyaninfarbstoff, ein Merocyaninfarbstoff, ein Oxonolfarbstoff, ein
Styrylfarbstoff oder ein Squariliumfarbstoff ist.

3. Antikörper-Farbstoffkonjugate gemäß Anspruch 1, **dadurch gekennzeichnet, dass** der Farbstoff ein Cyaninfarbstoff wie Carbocyanin, Dicarbocyanin oder Tricarbocyanin ist.

4. Antikörper-Farbstoffkonjugate gemäß den Ansprüchen 1 bis 3, **dadurch gekennzeichnet, dass** der Farbstoff -(F)ₙ der allgemeinen Formel I ein Cyaninfarbstoff der allgemeinen
Formel II 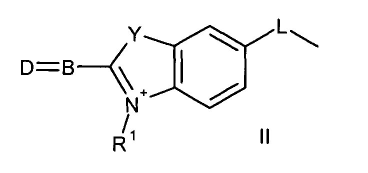 ist, in der
D für einen Rest III oder IV 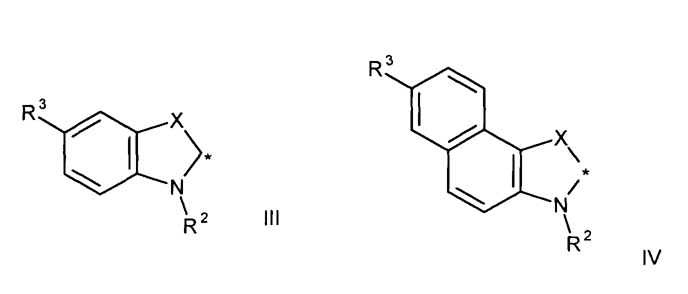 steht, wobei die mit einem Stern markierte Position die Verknüpfungsstelle mit dem Rest B bedeutet, und
B für die Gruppe V, VI, VII, VIII oder IX 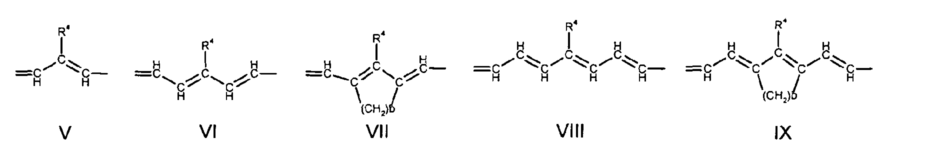 stehen kann, in denen
R¹ und R² C₁-C₄-Sulfoalkyl, eine gesättigte oder ungesättigte, verzweigte oder lineare
C₁-C₅₀-Alkylkette bedeutet, die gegebenfalls mit bis zu 15 Sauerstoffatomen, und/oder mit bis zu 3 Carbonylgruppen, und/oder mit bis zu 5 Hydroxygruppen substituiert sein kann,
R³ für die Gruppe -COOE¹, -CONE¹E², -NHCOE¹, -NHCONHE¹, -NE¹E², -OE¹, -OSO₃E¹, -SO₃E¹, -SO₂NHE¹, oder -E¹ steht, wobei
E¹ und E² unabhängig voneinander für ein Wasserstoffatom, C₁-C₄-Sulfoalkyl, gesättigtes oder ungesättigtes, verzweigtes oder geradkettiges C₁-C₅₀-Alkyl steht, dass gegebenfalls mit bis zu 15 Sauerstoffatomen, und/oder bis zu 3
Carbonylgruppen unterbrochen, und/oder mit bis zu 5 Hydroxygruppen substituiert sein kann,
R⁴ für ein Wasserstoffatom oder ein Fluor- Chlor, Brom- oder lodatom steht,
b für 2 oder 3 steht,
X und Y unabhängig voneinander für Sauerstoff, Schwefel oder die Gruppe =C(CH₃)₂ oder -(CH=CH)- steht,
und
L für eine direkte Bindung oder einen Linker, der eine geradkettige oder verzweigte Kohlenstoffkette mit bis zu 20 Kohlenstoffatomen, welche mit
einer oder mehreren -OH, -COOH, SO₃-Gruppen substituiert, und/oder gegebenenfalls ein oder mehrfach durch eine -O-, -S-, -CO-, -CS-, -CONH-, -NHCO-, -NHCSNH-, -SO₂-, PO₄⁻ oder eine -NH-Gruppen oder einen Arylring unterbrochen sein kann, steht.

5. Antikörper-Farbstoffkonjugate gemäß den Ansprüchen 1 bis 4, **dadurch gekennzeichnet, dass** als Antikörper die Antikörper L19 und E8 verwendet werden.

6. Antikörper-Farbstoffkonjugate gemäß den Ansprüchen 1 bis 5, **dadurch gekennzeichnet, dass** der Farbstoff im sichtbaren Spektralbereich des Lichtes ein optisches Signal induziert.

7. Antikörper-Farbstoffkonjugate gemäß den Ansprüchen 1 bis 5, **dadurch gekennzeichnet, dass** der Farbstoff erst unter Verwendung eines definierten Wellenlängenbereiches des sichtbaren oder nahinfraroten Lichtes ein Fluoreszenzsignal induziert.

8. Pharmazeutisches Mittel, umfassend ein oder mehrere Antikörper-Farbstoffkonjugate gemäß den Ansprüchen 1 bis 7, zur intraoperativen Darstellung der Randbereiche eines Krankheitsherdes.

9. Pharmazeutisches Mittel gemäß Anspruch 8, in Mischung mit geeigneten Lösungsmitteln, Puffern und/oder Trägerstoffen.

10. Verwendung der Antikörper-Farbstoffkonjugate und Mittel gemäß den Ansprüchen 1 bis 9 zur intraoperativen Darstellung von Krankheitsherden.

11. Verwendung der Antikörper-Farbstoffkonjugate gemäß den Ansprüchen 1 bis 9 zur intraoperativen Darstellung der Randbereiche eines Krankheitsherdes.

12. Verwendung der Antikörper-Farbstoffkonjugate gemäß den Ansprüchen 1 bis 9 zur mikro- und makroskopischen, intraoperativen Darstellung der Randbereiche eines Krankheitsherdes.

13. Verwendung der Antikörper-Farbstoffkonjugate gemäß den Ansprüchen 1 bis 7 zur Herstellung eines Mittels zur chirurgischen Behandlung von angiogeneseabhängigen Erkrankungen, wie malignen Tumoren und deren Metastasen, benignen Tumoren, präkanzeröse Gewebsveränderungen, Endometriose, Hämangiomen und extrauterinen Schwangerschaften.
